# EUROPÄISCHE PATENTANMELDUNG

(11) **EP 1 475 073 A2**
(43) Veröffentlichungstag der Anmeldung: **10.11.2004**
(21) Anmeldenummer: 04102814.3
(22) Anmeldetag: 20.07.2000
(51) Int. Cl.: A61K 7/06

(54) **Haarkosmetische Reinigungsmittel mit Wirkstoffkombinationen aus Gamma-Oryzanol und Calciumsalzen mit niedriger Metallionenkonzentration**

(30) Priorität: 22.07.1999 DE 19934385
(62) Teilanmeldung aus: 00115621.5
(71) Anmelder: Beiersdorf Aktiengesellschaft, 20245 Hamburg (DE)
(72) Erfinder: Argembeaux, Horst, 21465 Wentorf (DE); Koller, Andreas, 21035 Hamburg (DE)

(57) **Zusammenfassung**

Haarkosmetische Reinigungsmittel, die von folgenden Zubereitungen verschieden sind:
(1): 2,5 Gew.% Octylacrylamide/Acrylates/Butylaminoethyl Methacrylate Copolymer, 0,1 Gew.%, Gamma-Oryzanol, 0,05 Gew.% Calziumpanthotenat, 39 Gew.% Ethanol abs., Parfüm, Lösungsvermittler, Neutralisatioinsmittel/pH-Einstellung, Pflegestoffe q.s.,
(2): 2,5 Gew.% Octylacrylamide/Acrylates/Butylaminoethyl Methacrylate Copolymer, 0,05 Gew.%, Gamma-Oryzanol, 0,08 Gew.% Calziumpanthotenat, 39 Gew.% Ethanol abs., Parfüm, Lösungsvermittler, Neutralisatioinsmittel/pH-Einstellung, Pflegestoffe q.s.,
(3): 8 Gew.% PVPNA Copolymer, 0,1 Gew.%, Gamma-Oryzanol, 0,05 Gew.% Calziumpanthotenat, 39 Gew.% Ethanol abs., Parfüm, Lösungsvermittler, Neutralisatioinsmittel/pH-Einstellung, Pflegestoffe q.s.,
(4): 8 Gew.% PVPNA Copolymer, 0,05 Gew.%, Gamma-Oryzanol, 0,08 Gew.% Calziumpanthotenat, 39 Gew.% Ethanol abs., Parfüm, Lösungsvermittler, Neutralisatioinsmittel/pH-Einstellung, Pflegestoffe q.s.,
(5): 2,5 Gew.% Octylacrylamide/Acrylates/Butylaminoethyl Methacrylate Copolymer, 0,1 Gew.%, Gamma-Oryzanol, 0,05 Gew.% Calziumpanthotenat, Parfüm, Lösungsvermittler, Neutralisatioinsmittel/pH-Einstellung, Pflegestoffe q.s., Ethanol abs. ad 100,
(6): 2,5 Gew.% Octylacrylamide/Acrylates/Butylaminoethyl Methacrylate Copolymer, 0,05 Gew.%, Gamma-Oryzanol, 0,08 Gew.% Calziumpanthotenat, Parfüm, Lösungsvermittler, Neutralisatioinsmittel/pH-Einstellung, Pflegestoffe q.s., Ethanol abs. ad 100,
(7): 8 Gew.% PVP/VA Copolymer, 0,1 Gew.%, Gamma-Oryzanol, 0,05 Gew.% Calziumpanthotenat, Parfüm, Lösungsvermittler, Neutralisatioinsmittel/pH-Einstellung, Pflegestoffe q.s., Ethanol abs. ad 100,
(8): 8 Gew.% PVPNA Copolymer, 0,05 Gew.%, Gamma-Oryzanol, 0,08 Gew.% Calziumpanthotenat, Parfüm, Lösungsvermittler, Neutralisatioinsmittel/pH-Einstellung, Pflegestoffe q.s., Ethanol abs. ad 100,
(9): Zusammensetzung erhältlich durch Vereinigen nach Bsp. 2 der DE1617557 der Zubereitungen A aus 300 mg Raffinoselaurinsäureester, 30 mg Orizanol, 10 mg Lactotitan-Komplexverbindung, 50 mg Cerebiose, 100 ml Wasser und B aus 5 mg Vitamin B6, 10 mg Calziumpantothenat, 10 mg Allantoin, 1 mg Dihydronatriumacetoacetat, 10000 mg Ethylalkohol, 50 mg Natriumarinat, 10 mg Paraoxybenzoesäuremethylester, in geeigneter Menge Parfüm;
und dadurch gekennzeichnet sind, daß die Zubereitungen Gamma-Oryzanol und ein Calciumsalz oder mehrere Calciumsalze, ausgewählt aus der Gruppe gebildet von Calciumpanthotenat, Calciumchlorid und Calciumlactat, enthalten, wobei die Konzentration an in freier lonenform vorliegenden polyvalenten Metallkationen unter 0,004 M beträgt.

## Beschreibung

Die vorliegende Erfindung betrifft kosmetische Wirkstoffkombinationen und Zubereitungen, solche Wirkstoffkombinationen enthaltend. Insbesondere betrifft die vorliegende Erfindung haarkosmetische Reinigungszubereitungen oder haarkosmetische Reinigungsmittel mit einem Gehalt an Substanzen, die die Kopfhaut und/oder das Haar, aber auch die Zubereitungen selbst vor unerwünschten Oxidationsprozessen schützen. In einer bevorzugten Ausführungsform betrifft die vorliegende Erfindung Wirkstoffkombinationen und Zubereitungen damit, die dazu dienen, das Haar und die Kopfhaut zu pflegen.

Oxidative Prozesse schädigen Stoffe unterschiedlichster Natur (z.B. Haut, Haare und Wolle, aber auch Lacke und Kunststoffe, um nur einige zu nennen) zum Teil in erheblichem Maße. Die Stoffe ändern dabei ihre physikalischen und chemischen Eigenschaften: sie "altern". Zur Verzögerung oder sogar Inhibierung der Alterung von Stoffen werden im allgemeinen sogenannte Alterungsschutzmittel verwendet.

Insbesondere die schädigende Wirkung des ultravioletten Teils der Sonnenstrahlung auf organische, aber auch anorganische Stoffe ist allgemein bekannt. Für den Menschen sind dabei die Schädigung von Haut und Haaren und die Verzögerung oder Verhinderung derselben durch den Einsatz von Lichtschutzmitteln von besonderer Bedeutung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopf-haut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluß des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Sowohl UV-A- als auch UV-B-Strahlung haben einen schädigenden Einfluß auf das Haar, der sich beispielsweise darin äußert, daß bestimmte Aminosäuren wie Cystin und Methionin abgebaut oder Schwefel-Schwefel-Bindungen des Keratins gespalten werden, was im schlimmsten Fall eine Zerstörung des Haars zur Folge haben kann. Weiterhin stellen Haar und Kopfhaut Teile des Körpers dar, die aufgrund ihrer Position beim Aufenthalt im Freien einer erheblichen Menge an UV-Strahlung ausgesetzt sind.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Gewöhnlich werden dabei Wasserstoffperoxidkonzentrationen um 6% verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagiert und dabei unter Umständen eine Schädigung des Haares bewirken kann.

Oxidative Einflüsse, wie beispielsweise chemische Haarbehandlungen oder Sonnenlicht, setzen demnach die Festigkeit und die Elastizität des Haares herab und führen zu einer Zerstörung von Melanin. Augenscheinlich wird dies insbesondere bei dunkelhaarigen Personen, deren Haar im Sommer durch das intensive Sonnenlicht deutlich aufgehellt wird. Unter dem Bergriff "oxidativer Einfluß" ist im Sinne der vorliegenden Erfindung sowohl der Einfluß von oxidierend wirkenden Substanzen zu verstehen als auch die oxidative Wirkung von durch Strahlung, namentlich Licht, insbesondere UV-Licht, hervorgerufenen Folgeprodukten.

Ein Ziel der Haarpflege ist es, Kopfhaut und -haar vor oxidativen Einflüssen zu schützen und den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl gelten als Merkmale für natürliches, gesundes Haar.

Seit Ende des vergangenen Jahrhunderts werden Produkte zur Haarpflege gezielt entwickelt. Dies führte zu einer Vielzahl von Präparaten sowohl für die allgemeine Haarpflege als auch zur Behebung von Anomalien des Haares und der Kopfhaut. Im allgemeinen werden heutzutage Haarpflegekosmetika verwendet, welche entweder dazu bestimmt sind, nach dem Einwirken aus dem Haar wieder ausgespült zu werden, oder welche auf dem Haar verbleiben sollen. Letztere können so formuliert werden, daß sie nicht nur der Pflege des einzelnen Haars dienen, sondern auch das Aussehen einer Frisur insgesamt verbessern, beispielsweise dadurch, daß sie dem Haar mehr Fülle verleihen, die Frisur über einen längeren Zeitraum fixieren oder die Frisierbarkeit verbessern.

Die Verwendung von Antioxidantien, also Substanzen, die Oxidationsprozesse verhindern, in Kosmetika ist an sich bekannt. Antioxidantien, die in der Kosmetik Verwendung finden, sind beispielsweise Tocopherole, Gallensäurederivate, Sesamol und Flavonoide. Antioxidantien werden hauptsächlich als Schutzsubstanzen gegen den Verderb der sie enthaltenden Zubereitungen verwendet.

Auch Tocopherole, insbesondere Vitamin E, sind natürlich prinzipiell geeignet, Oxidationsprozesse zu verhindern, und finden dementsprechend häufig in Kosmetika Verwendung. Allerdings haben Tocopherole den Nachteil, daß sie im allgemeinen sehr reaktiv sind und daher zum Teil bereits in der Zubereitung abreagieren. Dies führt dazu, daß nur ein kleiner Teil der Einsatzmenge den zu schützenden Körperteil überhaupt erreicht, so daß die erzielte Wirkung weit hinter der erhofften zurückbleibt.

Aus der EP-A-0706366 sind Shampoozusammensetzungen bekannt, die eine Konzentration von 0,004 M bis 0,08 M an polyvalenten Metallkationen in freier lonenform aufweisen. Mit "M" wird, wie auch im folgenden die Molarität angegeben.

Kosmetische Zubereitungen dieser Art haben jedoch den Nachteil, sich durch Einfluß von Licht und/oder Wärme oder nach längerer Lagerung in ihrem Aussehen und/oder im Geruch nachteilig verändern zu können, weil ihre Konzentration an freien polyvalenten Metallkationen zu hoch ist.

Eine Aufgabe der vorliegenden Erfindung war, die Nachteile des Standes der Technik zu beseitigen. Insbesondere sollten kosmetische Wirkstoffe bzw. Zubereitungen, solche Wirkstoffe enthaltend, zur Verfügung gestellt werden, bei deren Verwendung die Schädigung der Kopfhaut und/oder des Haares durch oxidativen Einfluß gemindert, wenn nicht gänzlich verhindert werden kann. Diese kosmetischen Wirkstoffe bzw. Zubereitungen sollen in ihrer Wirksamkeit und ihrer kosmetischen Anmutung über einen längeren Zeitraum stabil bleiben.

Erfindungsgemäß wird diese Aufgabe gelöst und werden die Nachteile des Standes der Technik beseitigt.

Gegenstand der Erfindung sind haarkosmetische Reinigungsmittel, dadurch gekennzeichnet, daß sie Gamma-Oryzanol und ein Calciumsalz oder mehrere Calciumsalze, ausgewählt aus der Gruppe gebildet von Calciumpanthotenat, Calciumchlorid oder Calciumlactat, enthalten, wobei die Konzentration an in freier lonenform vorliegenden polyvalenten Metallkationen weniger als 0,004 M beträgt.

Gegenstand der Erfindung sind insbesondere haarkosmetische Reinigungsmittel, dadurch gekennzeichnet, daß sie Gamma-Oryzanol und ein Calciumsalz oder mehrere Calciumsalze, ausgewählt aus der Gruppe gebildet von Calciumpanthotenat, Calciumchlorid und Calciumlactat, und mindestens ein Komplexierungsmittel enthalten.

Gegenstand der Erfindung sind auch haarkosmetische Wirkstoffkombinationen, dadurch gekennzeichnet, daß sie γ Oryzanol und ein Calciumsalz oder mehrere Calciumsalze, ausgewählt aus der Gruppe gebildet von Calciumpanthotenat, Calciumchlorid und Calciumlactat, und mindestens ein Komplexierungsmittel enthalten.

Gegenstand der Erfindung ist weiterhin die Verwendung von Wirkstoffkombinationen und haarkosmetischen Reinigungsmitteln, die diese enthalten, bestehend aus Gamma-Oryzanol und einem Calciumsalz oder mehreren Calciumsalzen, ausgewählt aus der Gruppe gebildet von Calciumpanthotenat, Calciumchlorid und Calciumlactat, wobei die Konzentration an in freier lonenform vorliegenden polyvalenten Metallkationen weniger als 0,004 M beträgt, zum Schutz haarkosmetischer Zubereitungen und/oder der Kopfhaut und/oder des Haares vor unerwünschten Oxidationsprozessen.

Gegenstand der Erfindung ist weiterhin die Verwendung von Wirkstoffkombinationen und haarkosmetischen Reinigungsmitteln, die diese enthalten, bestehend aus Gamma-Oryzanol und einem Calciumsalz oder mehreren Calciumsalzen, ausgewählt aus der Gruppe gebildet von Calciumpanthotenat, Calciumchlorid und Calciumlactat, und mindestens einem Komplexierungsmittel zum Schutz haarkosmetischer Zubereitungen und/oder der Kopfhaut und/oder des Haares vor unerwünschten Oxidationsprozessen.

Bevorzugte haarkosmetische Zubereitungen sind Shampoos, die beispielsweise auch Konditionierhilfsmittel, insbesondere auch wasserlösliche Konditionierhilfsmittel enthalten können, wie z.B. im folgenden beschrieben.

Die erfindungsgemäßen Wirkstoffkombinationen und Zubereitungen, diese Wirkstoffkombinationen enthaltend, mindern die Schädigung der Kopfhaut und/oder des Haares durch oxidative Einflüsse besser als Wirkstoffe, Wirkstoffkombinationen und Zubereitungen des Standes der Technik. Insbesondere pflegen sie durch oxidativen Streß geschädigtes oder strapaziertes Haar bzw. beugen solchen Schäden vor. Insbesondere maskieren die Komplexierungsmittel die gegebenenfalls in Konzentrationen über 0,004 M in der Form freier lonen vorliegenden polyvalenten Metallkationen, z.B. Calciumionen oder Magnesiumionen, die zu unerwünschten Veränderungen von Aussehen und Geruch der Wirkstoffkombinationen und diese enthaltende Zubereitungen führen würden. In der maskierten bzw. komplexierten Form führen die genannten Metallionen überraschenderweise nicht zu den geschilderten Nachteilen.

Gamma-Oryzanol ist in der Literatur beschrieben (CAS-Nr.: 11042-64-1 bzw. 12738-23-7). Gamma-Oryzanol ist keine einheitliche Verbindung, sondern ein Gemisch verschieden strukturierter Ferulasäureester. Gamma-Oryzanol besteht hauptsächlich aus den Estern der Ferulasäure mit den Triterpenalkoholen Cycloartenol und 24-Methylencylcloartenol. Ferner enthält es geringe Mengen an Estern mit Sterolen, namentlich mit Camesterol, Stigmasterol und β-Sitosterol.

Gamma-Oryzanol ist im Handel erhältlich, beispielsweise unter dem Handelsnamen Gamma-Oryzanol (Lieferant: Jan Dekker oder Henry Lamotte, DE).

Vorteilhaft enthalten erfindungsgemäße Zubereitungen 0,001 bis 5,0 Gew.-% Gamma-Oryzanol, bevorzugt 0,01 bis 2,0 Gew.-%, jeweils bezogen auf das Gesamtgewicht der Zubereitungen.

Vorteilhaft enthalten erfindungsgemäße Zubereitungen 0,001 bis 0,05 Gew.-% eines oder mehrerer Calciumsalze, bevorzugt 0,02 bis 0,05 Gew.-%, jeweils bezogen auf das Gesamtgewicht der Zubereitungen.

Als Calciumsalz wird Calciumlactat bevorzugt. Die Calciumsalze sind im Handel erhältlich.

Geeignet sind alle Komplexierungsmittel, die mehrwertige Metallkationen komplexieren und die Konzentration freier, bzw. in freier lonenform vorliegender polyvalenter Metallkationen, insbesondere Calciumionen und/oder Magnesiumionen, in den erfindungsgemäßen Zubereitungen senken, insbesondere unter die Konzentration von 0,004 M.

Als Komplexierungsmittel werden Ethylenbis(oxyethylennitrilo)-tetraessigsäure (EGTA), Ethylendiamintetraessigsäure oder Iminodibernsteinsäure bevorzugt, bzw. deren Salze, insbesondere wasserlösliche Salze, z.B. die Natriumsalze oder Kaliumsalze, wobei jeweils eine oder mehrere Carbonsäuregruppen in der Salzform vorliegen können.

Weiterhin können Komplexbildner vorteilhaft aus der Gruppe der üblichen Verbindungen gewählt werden, z.B. indem mindestens eine Substanz aus der Gruppe bestehend aus Weinsäure und deren Anionen, Citronensäure und deren Anionen, Aminopolycarbonsäuren und deren Anionen (wie beispielsweise Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen) gewählt wird.

Es kann angenommen werden, wobei aber diese Annahme noch nicht in allen Details bestätigt werden konnte, daß ein Anteil der theoretisch in den Zubereitungen verfügbaren polyvalenten Metallkationen, beispielsweise Calcium- oder Magnesiumionen, in der Form des erfindungsgemäßen Komplexes mit Gamma-Oryzanol vorliegt und ein weiterer Anteil dieser Metallkationen erfindungsgemäß, insbesondere von dem Komplexierungsmittel maskiert wird und die Konzentration polyvalenter Metallkationen, die in freier lonenform vorliegen, unter 0,004 M liegt.

Das zur Herstellung der Zubereitungen verwendete Wasser ist vorzugsweise demineralisiert oder vollentsalzt (VES) oder besitzt nur eine geringe Wasserhärte bzw. geringe Konzentration an Calciumionen oder Magnesiumionen, falls Leitungswasser verwendet wird. Zweckmäßigerweise wird insbesondere in diesen Fällen die Konzentration an freien polyvalenten Metallkationen bezüglich der erfindungsgemäßen geringeren Konzentration von 0,004 M überprüft, beispielsweise durch Messungen mit einer kationensensitiven Elektrode.

Korrekturen können immer, falls zweckmäßig oder erforderlich, durch den Fachmann in bekannter Weise vorgenommen werden, beispielsweise durch eine angepaßte und z.B. durch eine solche Messung kontrollierte, höhere Dosierung des Komplexierungsmittels oder aber eine geringere Dosierung, falls die Verwendung eines Komplexierungsmittels erforderlich sein sollte, da auch eine geringere erfindungsgemäße Dosierung von polyvalenten, freien Metallkationen, insbesondere Calciumionen, unter der Obergrenze einer Konzentration von 0,004 M vorgenommen und gegebenenfalls meßtechnisch eingestellt und kontrolliert werden kann.

Vorteilhaft enthalten erfindungsgemäße Zubereitungen 0,02 - 2,00 Gew.-% eines oder mehrerer Komplexierungsmittel, bevorzugt 0,05 - 1,0 Gew.-%, jeweils bezogen auf das Gesamtgewicht der Zubereitungen.

Die Komplexierungsmittel sind ebenfalls bekannt und im Handel erhältlich.

In den erfindungsgemäßen Zubereitungen beträgt die Konzentration an polyvalenten Metallkationen, insbesondere Calciumionen, die in freier lonenform vorliegen, beispielsweise 0,000001 M - 0,0039 M, vorzugsweise 0,00001 M - 0,0039 M, insbesondere 0,0001 M - 0,0038 M.

Die haarkosmetischen Reinigungsmittel und Zubereitungen, die erfindungsgemäße Wirkstoffkombinationen enthalten, sind topische Zubereitungen. Diese können wie üblich zusammengesetzt sein und zur Behandlung und der Pflege der Kopfhaut und/oder der Haare oder als Lichtschutzpräparat dienen. Zur Anwendung werden die erfindungsgemäßen Zubereitungen in der für Kosmetika und Haarreinigungsmittel üblichen Weise auf die Kopfhaut und die Haare in ausreichender Menge aufgebracht.

Vorteilhaft können Zubereitungen im Sinne der vorliegenden Erfindung als Emulsionen oder Lösungen vorliegen.

Die Mittel gemäß der Erfindung können beispielsweise als aus Quetschflaschen oder durch eine Pump- oder Sprühvorrichtung dosierbare Präparate vorliegen, jedoch auch insbesondere in Form eines aus normalen Flaschen und Behältern auftragbaren Mittels.

Vorteilhaft enthalten erfindungsgemäße Zubereitungen neben einem wirksamen Gehalt an erfindungsgemäßen Wirkstoffkombinationen ferner übliche Wirk-, Inhalts-, Zusatzund/oder Hilfsstoffe.

Erfindungsgemäße Zubereitungen, die haarkosmetische Reinigungszubereitungen für das Haar bzw. die Kopfhaut darstellen, können in flüssiger oder fester Form vorliegen. Sie enthalten vorzugsweise mindestens eine anionische, nicht-ionische oder amphotere oberflächenaktive Substanz oder Gemische daraus, gegebenenfalls einen Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen, insbesondere aber zwischen 1 und 50 Gew.-%.

Insbesondere können erfindungsgemäße wäßrige kosmetische Reinigungsmittel oder für die wäßrige Reinigung bestimmte wasserarme oder wasserfreie Reinigungsmittelkonzentrate anionische, nichtionische und/oder amphotere Tenside enthalten, beispielsweise herkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate enthalten.

Anionische Tenside werden vorzugsweise in Konzentrationenzwischen 5 Gew.-% und 20 Gew.-% eingesetzt. In Frage kommen z.B. Sodium Laureth Sulfate wie es unter der Bezeichnung Texapon N 70 von der Fa. Henkel angeboten wird oder Disodium Laureth Sulfosuccinate wie es unter der Bezeichnung Rewopol SBFA 30 von der Fa. Witco angeboten wird. Nichtionische Tenside werden vorzugsweise in Konzentrationen von 1 Gew.-% bis 10 Gew.-% eingesetzt. Beispiele sind Decyl Glucoside wie es unter der Bezeichnung Oramix NS 10 von der Fa. Seppic angeboten wird oder Polysorbate 80 wie es unter der Bezeichnung Tween 80 von der Fa. ICI angeboten wird. Amphotere Tenside werden vorzugsweise in Konzentrationen von 1 Gew.-% bis 10 Gew.-% eingesetzt. Beispiele sind Cocamidopropyl Betaine wie es als Tego Betain von der Fa. Goldschmidt angeboten wird oder Sodium Cocoamphoacetate wie es unter der Bezeichnung Miranol Ultra von der Fa. Rhone Poulenc angeboten wird.

Die Prozentangaben beziehen sich auf das Gesamtgewicht der Zubereitungen.

Weiterhin können in den haarkosmetischen Reinigungsmitteln Konditionierhilfsmittel, insbesondere wasserlösliche Konditioniermittel, z.B. kationische Konditioniermittel, enthalten sein, z.B. in Mengen von 0,001 bis 10 Gew.-%, bezogen auf das Gesamtgewicht der Zubereitungen. Zu den bevorzugten Konditionierhilfsmitteln gehören polymere quaternäre Verbindungen (Quats). Polymere Quats werden vielfach in Shampoos z.B. mit einer Konzentration von 0,01 bis 2 Gew.-% eingesetzt. Dazu gehören Polyquaternium-10 wie es unter der Bezeichnung Polymer JR 400 von der Fa. Amerchol angeboten wird oder Hydroxypropyl Guar Hydroxypropyltrimonium Chloride wie es mit der Bezeichnung Jaguar C 162 von der Fa. Rhone-Poulenc angeboten wird.

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Der Wassergehalt der Zubereitungen beträgt z.B. 20 - 99 Gew.-%, vorzugsweise 50 bis 95 Gew.-%, insbesondere 55 bis 90 Gew.-%, jeweils bezogen auf das Gesamtgewicht der Zubereitung.

Erfindungsgemäß können als weitere Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,001 bis 2 Gew.-%, vorzugsweise 0,01 bis 1 Gew.-%, jeweils bezogen auf das Gesamtgewicht der Zubereitung.

Vorteilhaft werden weitere Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, ungesättigte Fettsäuren und deren Derivate (z.B. γ-Linolensäure, Linolsäure, Ölsäure), Folsäure und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Vorteilhaft können erfindungsgemäße Zubereitungen außerdem Substanzen enthalten, die UV-Strahlung im UV-B-Bereich absorbieren, wobei die Gesamtmenge der Filtersubstanzen z.B. 0,001 Gew.-% bis 30 Gew.-%, vorzugsweise 0,05 bis 10 Gew.-%, insbesondere 0,1 bis 1,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut, insbesondere die Kopfhaut dienen.

Enthalten die erfindungsgemäßen Emulsionen UV-B-Filtersubstanzen, können diese vorteilhaft wasserlöslich sein. Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Es kann auch von Vorteil sein, erfindungsgemäße Zubereitungen mit UV-A-Filtern zu versetzen, die bisher üblicherweise in kosmetischen Zubereitungen enthalten sind. Es können die für die UV-B-Kombination verwendeten Mengen eingesetzt werden.

Das Gewichtsverhältnis der Wirkstoffe Oryzanol/Calciumsalze zueinander in den Kombinationen kann in einem breiten Bereich variiert werden und kann beispielsweise 100 : 1 bis 1 : 100, vorzugsweise 10 : 1 bis 1 : 10 betragen, aber auch 1 : 1 betragen.

Das Gewichtsverhältnis der Wirkstoffe Calciumsalze / Komplexbildner zueinander in den Kombinationen kann in einem breiten Bereich variiert werden und kann beispielsweise 100 : 1 bis 1 : 100, vorzugsweise 10 : 1 bis 1 : 10 betragen, aber auch 1 : 1 betragen.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat oder Phosphorsäure Phosphat-Puffergemischen. Vorzugsweise liegt der pH-Wert unter 10, z.B. im Bereich von 4-8, insbesondere im Bereich von 5 - 7.

Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen.

Die folgenden Beispiele verdeutlichen die Erfindung.

Als Gamma-Oryzanol wird in den Beispielen das Handelsprodukt Gamma-Oryzanol der Firma Jan Dekker, DE, verwendet.

Als Iminodibernsteinsäure-Na-salz wurde das Tetranatriumsalz verwendet.

Die Mengenangaben in den Beispielen sind Gew.-%.

**Beispiele 1 - 3**

| Conditioner-Shampoo mit Perlglanz | | | |
|---|---|---|---|
| | **1** | **2** | **3** |
| Polyquaternium-10 | 0,5 | 0,5 | 0,5 |
| Natriumlaurethsulfat | 9,0 | 9,0 | 9,0 |
| Cocoamidopropylbetain | 2,5 | 2,5 | 2,5 |
| Perlglanzmittel | 2,0 | 2,0 | 2,0 |
| Gamma-Oryzanol | 0,01 | 0,15 | 0,05 |
| Calziumlactat | 0,015 | 0,05 | 0,01 |
| Dinatrium EDTA | 0,1 | 0,2 | 0,15 |
| Konservierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |

Der pH-Wert wird auf 6 eingestellt.

**Beispiele 4 - 6**

| klares Conditioner-Shampoo | | | |
|---|---|---|---|
| | **4** | **5** | **6** |
| Polyquaternium-10 | 0,5 | 0,5 | 0,5 |
| Natriumlaurethsulfat | 9,0 | 9,0 | 9,0 |
| Cocoamidopropylbetain | 2,5 | 2,5 | 2,5 |
| Gamma-Oryzanol | 0,25 | 0,2 | 0,15 |
| Calziumchlorid | 0,015 | 0,03 | 0,05 |
| Iminodibernsteinsäure, Na-Salz | 0,2 | 0,3 | 0,8 |
| Konservierungsmittel, Parfüm, Verdicker, pH-Einstellung und Lösungsvermittler | q.s. | q.s. | q.s. |
| Wasser, VES | ad 100,0 | ad 100,0 | ad 100,0 |

Der pH-Wert wird auf 6 eingestellt.

**Beispiele 7 - 9**

| klares Light-Shampoo mit Volumeneffekt | | | |
|---|---|---|---|
| | **7** | **8** | **9** |
| Natriumlaurethsulfat | 10,0 | 10,0 | 10,0 |
| Cocoamidopropylbetain | 2,5 | 2,5 | 2,5 |
| Gamma-Oryzanol | 0,01 | 0,02 | 0,05 |
| Calziumchlorid | 0,01 | 0,008 | 0,05 |
| Dinatrium EDTA | 0,2 | 0,15 | 0,7 |
| Konservierungsmittel, Parfüm, Verdicker, pH-Einstellung und Lösungsvermittler | q.s. | q.s. | q.s. |
| Wasser, VES | ad 100,0 | ad 100,0 | ad 100,0 |

Der pH-Wert wird auf 5,5 eingestellt.

**Beispiele 10 -12**

| klares Conditioner-Shampoo | | | |
|---|---|---|---|
| | **10** | **11** | **12** |
| Polyquaternium-10 | 0,5 | 0,5 | 0,5 |
| Natriumlaurethsulfat | 9,0 | 9,0 | 9,0 |
| Cocoamidopropylbetain | 2,5 | 2,5 | 2,5 |
| Gamma-Oryzanol | 0,25 | 0,2 | 0,15 |
| Calziumchlorid | 0,0015 | 0,003 | 0,005 |
| Konservierungsmittel, Parfüm, Verdicker, pH-Einstellung und Lösungsvermittler | q.s. | q.s. | q.s. |
| Wasser, VES | ad 100,0 | ad 100,0 | ad 100,0 |

Der pH-Wert wird auf 6 eingestellt.

## Patentansprüche

1. Haarkosmetische Reinigungsmittel, die von folgenden Zubereitungen verschieden sind:
(1): 2,5 Gew.% Octylacrylamide/Acrylates/Butylaminoethyl Methacrylate Copolymer, 0,1 Gew.%, Gamma-Oryzanol, 0,05 Gew.% Calziumpanthotenat, 39 Gew.% Ethanol abs., Parfüm, Lösungsvermittler, Neutralisatioinsmittel/pH-Einstellung, Pflegestoffe q.s.,
(2): 2,5 Gew.% Octylacrylamide/Acrylates/Butylaminoethyl Methacrylate Copolymer, 0,05 Gew.%, Gamma-Oryzanol, 0,08 Gew.% Calziumpanthotenat, 39 Gew.% Ethanol abs., Parfüm, Lösungsvermittler, Neutralisatioinsmittel/pH-Einstellung, Pflegestoffe q.s.,
(3): 8 Gew.% PVP/VA Copolymer, 0,1 Gew.%, Gamma-Oryzanol, 0,05 Gew.% Calziumpanthotenat, 39 Gew.% Ethanol abs., Parfüm, Lösungsvermittler, Neutralisatioinsmittel/pH-Einstellung, Pflegestoffe q.s.,
(4): 8 Gew.% PVP/VA Copolymer, 0,05 Gew.%, Gamma-Oryzanol, 0,08 Gew.% Calziumpanthotenat, 39 Gew.% Ethanol abs., Parfüm, Lösungsvermittler, Neutralisatioinsmittel/pH-Einstellung, Pflegestoffe q.s.,
(5): 2,5 Gew.% Octylacrylamide/Acrylates/Butylaminoethyl Methacrylate Copolymer, 0,1 Gew.%, Gamma-Oryzanol, 0,05 Gew.% Calziumpanthotenat, Parfüm, Lösungsvermittler, Neutralisatioinsmittel/pH-Einstellung, Pflegestoffe q.s., Ethanol abs. ad 100,
(6): 2,5 Gew.% Octylacrylamide/Acrylates/Butylaminoethyl Methacrylate Copolymer, 0,05 Gew.%, Gamma-Oryzanol, 0,08 Gew.% Calziumpanthotenat, Parfüm, Lösungsvermittler, Neutralisatioinsmittel/pH-Einstellung, Pflegestoffe q.s., Ethanol abs. ad 100,
(7): 8 Gew.% PVP/VA Copolymer, 0,1 Gew.%, Gamma-Oryzanol, 0,05 Gew.% Calziumpanthotenat, Parfüm, Lösungsvermittler, Neutralisatioinsmittel/pH-Einstellung, Pflegestoffe q.s., Ethanol abs. ad 100,
(8): 8 Gew.% PVP/VA Copolymer, 0,05 Gew.%, Gamma-Oryzanol, 0,08 Gew.% Calziumpanthotenat, Parfüm, Lösungsvermittler, Neutralisatioinsmittel/pH-Einstellung, Pflegestoffe q.s., Ethanol abs. ad 100,
(9): Zusammensetzung erhältlich durch Vereinigen nach Bsp. 2 der DE1617557 der Zubereitungen A aus 300 mg Raffinoselaurinsäureester, 30 mg Orizanol, 10 mg Lactotitan-Komplexverbindung, 50 mg Cerebiose, 100 ml Wasser und B aus 5 mg Vitamin B6, 10 mg Calziumpantothenat, 10 mg Allantoin, 1 mg Dihydronatriumacetoacetat, 10000 mg Ethylalkohol, 50 mg Natriumarinat, 10 mg Paraoxybenzoesäuremethylester, in geeigneter Menge Parfüm;
und **dadurch gekennzeichnet sind, daß** die Zubereitungen Gamma-Oryzanol und ein Calciumsalz oder mehrere Calciumsalze, ausgewählt aus der Gruppe gebildet von Calciumpanthotenat, Calciumchlorid und Calciumlactat, enthalten, wobei die Konzentration an in freier lonenform vorliegenden polyvalenten Metallkationen unter 0,004 M beträgt.

2. Verwendung von Wirkstoffkombinationen und haarkosmetischen Reinigungsmitteln, die diese enthalten, bestehend aus Gamma-Oryzanol und einem Calciumsalz oder mehreren Calciumsalzen, ausgewählt aus der Gruppe gebildet von Calciumpanthotenat, Calciumchlorid und Calciumlactat, wobei die Konzentration an in freier lonenform vorliegenden polyvalenten Metallkationen weniger als 0,004 M beträgt, zum Schutz haarkosmetischer Zubereitungen und/oder der Kopfhaut und/oder des Haares vor unerwünschten Oxidationsprozessen, wobei die Wirkstoffkombinationen und haarkosmetischen Reinigungsmittel von folgenden Zubereitungen (gemäß EP 937450 A1 [(1)-(8)] und DE1617557 (9)} verschieden sind:
(1): 2,5 Gew.% Octylacrylamide/Acrylates/Butylaminoethyl Methacrylate Copolymer, 0,1 Gew.%, Gamma-Oryzanol, 0,05 Gew.% Calziumpanthotenat, 39 Gew.% Ethanol abs, Parfüm, Lösungsvermittler, Neutralisatioinsmittel/pH-Einstellung, Pflegestoffe q.s.,
(2): 2,5 Gew.% Octylacrylamide/Acrylates/Butylaminoethyl Methacrylate Copolymer, 0,05 Gew.%, Gamma-Oryzanol, 0,08 Gew.% Calziumpanthotenat, 39 Gew.% Ethanol abs, Parfüm, Lösungsvermittler, Neutralisatioinsmittel/pH-Einstellung, Pflegestoffe q.s.,
(3): 8 Gew.% PVP/VA Copolymer, 0,1 Gew.%, Gamma-Oryzanol, 0,05 Gew.% Calziumpanthotenat, 39 Gew.% Ethanol abs, Parfüm, Lösungsvermittler, Neutralisatioinsmittel/pH-Einstellung, Pflegestoffe q.s.,
(4): 8 Gew.% PVP/VA Copolymer, 0,05 Gew.%, Gamma-Oryzanol, 0,08 Gew.% Calziumpanthotenat, 39 Gew.% Ethanol abs, Parfüm, Lösungsvermittler, Neutralisatioinsmittel/pH-Einstellung, Pflegestoffe q.s.,
(5): 2,5 Gew.% Octylacrylamide/Acrylates/Butylaminoethyl Methacrylate Copolymer, 0,1 Gew.%, Gamma-Oryzanol, 0,05 Gew.% Calziumpanthotenat, Parfüm, Lösungsvermittler, Neutralisatioinsmittel/pH-Einstellung, Pflegestoffe q.s., Ethanol abs. ad 100,
(6): 2,5 Gew.% Octylacrylamide/Acrylates/Butylaminoethyl Methacrylate Copolymer, 0,05 Gew.%, Gamma-Oryzanol, 0,08 Gew.% Calziumpanthotenat, Parfüm, Lösungsvermittler, Neutralisatioinsmittel/pH-Einstellung, Pflegestoffe q.s., Ethanol abs. ad 100,
(7): 8 Gew.% PVP/VA Copolymer, 0,1 Gew.%, Gamma-Oryzanol, 0,05 Gew.% Calziumpanthotenat, Parfüm, Lösungsvermittler, Neutralisatioinsmittel/pH-Einstellung, Pflegestoffe q.s., Ethanol abs. ad 100,
(8): 8 Gew.% PVP/VA Copolymer, 0,05 Gew.%, Gamma-Oryzanol, 0,08 Gew.% Calziumpanthotenat, Parfüm, Lösungsvermittler, Neutralisatioinsmittel/pH-Einstellung, Pflegestoffe q.s., Ethanol abs. ad 100,
(9): Zusammensetzung erhältlich durch Vereinigen nach Bsp. 2 der DE1617557 der Zubereitungen A aus 300 mg Raffinoselaurinsäureester, 30 mg Orizanol, 10 mg Lactotitan-Komplexverbindung, 50 mg Cerebiose, 100 ml Wasser und B aus 5 mg Vitamin B6, 10 mg Calziumpantothenat, 10 mg Allantoin, 1 mg Dihydronatriumacetoacetat, 10000 mg Ethylalkohol, 50 mg Natriumarinat, 10 mg Paraoxybenzoesäuremethylester, in geeigneter Menge Parfüm.

3. Mittel oder Verwendung gemäß Anspruch 1 oder 2, **dadurch gekennzeichnet, daß** sie 0,02 bis 2,00 Gew.-% eines oder mehrerer Komplexierungsmittel, bevorzugt 0,05 bis 1,0 Gew.-%, jeweils bezogen auf das Gesamtgewicht der Mittel, enthalten.

4. Mittel oder Verwendung gemäß Anspruch 1, 2 oder 3, **dadurch gekennzeichnet, daß** sie 0,001 bis 5,0 Gew.-% Gamma-Oryzanol, bevorzugt 0,01 bis 2,0 Gew.-%, jeweils bezogen auf das Gesamtgewicht der Mittel, enthalten.

5. Mittel oder Verwendung nach einem der vorangehenden Ansprüche, **dadurch gekennzeichnet, daß** zusätzlich weitere Tenside und/oder kosmetische oder dermatologische Hilfs-, Zusatz- und/oder Wirkstoffe enthalten sind.

6. Mittel oder Verwendung nach einem der vorangehenden Ansprüche, **dadurch gekennzeichnet, daß** sie 0,001 bis 0,05 Gew.-% eines oder mehrerer Calciumsalze, bevorzugt 0,02 bis 0,05 Gew.-%, jeweils bezogen auf das Gesamtgewicht der Mittel, enthalten.
